# EUROPÄISCHE PATENTANMELDUNG

(11) **EP 2 478 889 A2**
(43) Veröffentlichungstag der Anmeldung: **25.07.2012**
(21) Anmeldenummer: 12000448.6
(22) Anmeldetag: 24.01.2012
(51) Int. Cl.: A61K 6/00

(54) **Medizinisches Präparat zur Behandlung der Zahnwurzel**

(30) Priorität: 24.01.2011 DE 102011009305
(71) Anmelder: Winkelmann, Wolfgang, 72076 Tübingen (DE)
(72) Erfinder: Winkelmann, Wolfgang, 72076 Tübingen (DE)
(74) Vertreter: Wüstefeld, Regine Marie

(57) **Zusammenfassung**

Die Erfindung betrifft die Verwendung von ozonisiertem Pflanzenöl und/oder dessen Bestandteilen oder von Jod, insbesondere PVP-Jod, als keimtötendes Mittel zur Herstellung eines medizinischen Präparats zur Behandlung der Zahnwurzel, insbesondere bei Gangränen. Als Bestandteil des Pflanzenöls kann auch die reine Ölsäure gesehen werden.

Die Erfindung betrifft außerdem ein medizinisches Präparat zur Behandlung von Pulpengangränen in Form von vorzugsweise längenkalibrierten Papierspitzen, die eine Beschichtung von ozonisiertem Pflanzenöl und/oder dessen Bestandteilen oder von Jod, insbesondere PVP-Jod, aufweisen.

Die Erfindung betrifft des weiteren die Verwendung des genannten medizinischen Präparats als temporäre Einlage in das Endodont, besonders zur Abtötung von Keimen, ausgewählt aus den Bakterienstämmen Enterococcus faecalis und Candida albicans.

## Beschreibung

Die Erfindung betrifft die Verwendung von ozonisiertem Pflanzenöl und/oder dessen Bestandteilen oder von Jod, insbesondere PVP-Jod, als keimtötendem Mittel zur Herstellung eines medizinischen Präparats für eine spezielle Anwendung, das medizinische Präparat selbst sowie dessen bevorzugtes Anwendungsgebiet.

Bei einem Zahn sitzt der nach außen sichtbare Zahnschmelz schützend über der Pulpakammer, welche das Zahnmark enthält. Diese Pulpakammer läuft in die beiden Äste der Zahnwurzeln aus, die als Wurzelkanäle bezeichnet werden. Dieses Wurzelkanalsystem ist das Endodont. Hier unterscheidet man zunächst im Bereich der Verästelung das koronale Drittel, an das sich das mittlere Drittel anschließt, um schließlich im Bereich des apikalen Drittels in der Wurzelspitze (Apex) zu enden.

Das Zahnmark kann sich entzünden. Solche Entzündungen des Zahnmarks werden Pulpitis genannt. Eine Ursache für eine solche Pulpitis kann Karies sein. Dieser ist dann verantwortlich für einen sogenannten kariösen Defekt, durch den dann Krankheitskeime in die sonst gehütete Pulpakammer eindringen können.

Wenn der Entzündungsvorgang nicht unterbrochen wird, kann es zu einer irreversiblen Pulpitis kommen. Erreichen die Bakterien die Pulpa, entstehen lokale Abs-zesse und die Pulpa stirbt ab. Es entsteht das Pulpengangrän. Kann der Zahn noch restauriert werden, geschieht dies über eine Wurzelbehandlung.

Bei der Behandlung eines devitalen Zahnes besteht grundsätzlich das Problem, daß der oder die Wurzelkanäle zuerst gründlich desinfiziert werden müssen, bevor diese abgefüllt und bakteriendicht versiegelt werden können. Die möglichst vollständige Eliminierung aller im Kanalsystem anwesenden pathogenen Keime (Anaerobier) ist Voraussetzung für das Gelingen aller nachfolgenden Behandlungsschritte und den gesamten Erfolg einer Wurzelbehandlung.

Ziel einer jeden Wurzelbehandlung ist es dann, nach Entfernen der Kronenpulpa das System der Wurzelkanäle zu reinigen und von eingedrungenen Keimen zu befreien. Abgesehen von mechanischen Problemen bei der Aufbereitung und mechanischen Reinigung liegt die Schwierigkeit einer vollständigen Eliminierung aller Keime u.a. auch darin, daß der Wurzelkanal vor allem im unteren apikalen Drittel zahlreiche sog. "Ramifikationen", d.h. Verästelungen, aufweist. Es handelt sich um ein komplexes "Kanalsystem" mit zahlreichen Nebenkanälchen, von dem nur ein Teil mechanisch instrumentell gereinigt werden kann. D.h. insbesondere die Nebenkanälchen können mit den Aufbereitungsinstrumenten, wie z.B. Nadeln, nicht erreicht werden.

Grundsätzlich wird bei der Reinigung zunächst die Kanalpulpa mit der Pulpanadel, einer Nervnadel, entfernt. Dabei besteht zusätzlich die Gefahr, daß die Pulpanadel über die Wurzelspitze hinausgeschoben wird und in das Knochengewebe eindringt. Die regelmäßig bei einer solchen Entzündung vorhandenen Bakterien können dann bis in das Knochengewebe eindringen.

Bei jedem der genannten Arbeitsschritte und insbesondere nach der Reinigung der Wurzelkanäle wird ein Desinfektionsschritt vorgenommen. Dazu werden die Wurzelkanäle mit einer desinfizierenden Lösung ausgespült. Dieses Spülen hat zusätzlich reinigende Wirkung, um eventuelle weitere Verunreinigungen aus den Wurzelkanälen zu entfernen. Für das Spülen mit der desinfizierenden Lösung sind Lösungen von NaOCl, 3%-ige H₂O₂-Lösung, EDTA, Chlorhexidin (CHX) oder Zitronensäure im Stand der Technik bekannt.

Anschließend werden die Wurzelkanäle mit Papierspitzen sorgfältig getrocknet, damit sie die Wurzeifüllung aufnehmen können, ohne eine weitere Entzündung nach sich zu ziehen. Nicht vollständig entfernte Bakterienherde sind immer wieder Auslöser für eine weitere schmerzhafte Entzündung.

Da eine mechanische Reinigung in den genannten Ramifikationen in Form der Verästelungen und Nebenkanäle aufgrund der geschilderten anatomischen Verhältnisse also grundsätzlich nicht vollständig möglich ist, ist es das Bemühen im Stand der Technik, die in den Ramifikationen befindlichen oder verbliebenden Keime möglichst effektiv mit antibakteriellen Substanzen zu bekämpfen. Diese Substanzen sollen in die feinen Kanäle eindringen oder eindiffundieren können, um die dort befindlichen oder verbliebenen Erreger zu eliminieren, und sie sollen keine unerwünschten, nicht abbaubaren Rückstände hinterlassen.

Bei dieser Aufgabe ist bisher das gleichzeitige medizinische Ziel, keine weiteren Beschwerden beim Patienten und keine Nachteile für das umgebende Gewebe zu verursachen, nur schwer zu erreichen.

Bei älteren Präparaten sind z.B. Formaldehyd oder ChKM gemäß der "DGZMK"-Empfehlung zu nennen. Unter ChKM wird eine Chlorphenol-Kampfer-Menthol-Lösung verstanden, wie sie von der SPEIKO-Dr. Speier GmbH im Handel erhältlich ist. Diese Lösung wird insbesondere als medikamentöse Einlage des mit den Bakterien infizierten Wurzelkanals verwendet. Der Hersteller gibt selbst an, daß diese ChKM-Lösung nicht bei parodontologisch behandlungsbedürftigen Zähnen verwendet werden sollte. Bereits 1988 haben Chang und Messer in verschiedenen Untersuchungen in Laborversuchen festgestellt, daß die Zytotoxizität solcher Präparate größer sei als deren antibakterielle Wirkung (siehe in DAZ FORUM 86, 4. Quartal 2005).

Es ist des weiteren bekannt, zum Abtöten von Keimen bei Bedarf eine temporäre, sogenannte "medizinische Einlage" vorzusehen, die für einige Tage, manchmal auch länger und bis zu mehreren Wochen, in den Wurzelkmälen verbleiben muß. Das Standardmittel hierfür ist derzeit vor allem Calciumhydroxid (Ca(OH)₂), das in sahnig-pastöser Form in den Wurzelkanal eingebracht wird und bei stärkerer bakterieller Kontamination eine Art "Goldstandard" darstellt. Durch seinen stark alkalischen pH-Wert von ca. 11,0 zeigt Ca(OH)₂ eine herausragende desinfizierende Wirkung für fast alle in diesem Bereich auftretenden Bakterien und Keime.

Es ist bekannt, für das Einbringen der in die zuvor aufbereiteten Kanäle ein Lentulo zu verwenden. Darunter versteht man eine sehr dünne, zerbrechliche Rührspindel.

Die Gefahr, daß sich der Lentulo im Kanal verhakt und das Bruchstück dann im unteren Teil des Kanals unerreichbar steckenbleibt, ist dabei sehr groß. Ein solches Bruchfragment läßt sich in der Regel nicht mehr aus diesem unteren Teil des Wurzelkanals entfernen. Als Konsequenz wird dadurch eine Weiterbehandlung des Zahnes zu seiner Erhaltung unmöglich.

Alternativ dazu ist es bekannt, die Calciumhydroxid-Paste mittels Watte in den Wurzelkanal hineinzustopfen. Diese Methode hat wiederum den Nachteil und birgt somit die Gefahr, daß die Paste nicht ausreichend tief in den Wurzelkanal eingebracht wird, wodurch sie nicht ausreichend wirksam sein kann, oder daß sie zu tief appliziert wird.

Letzteres bedeutet, daß sie über den Apex hinausgerührt oder -gepreßt wird, und aufgrund ihres stark alkalischen pH-Wertes im umgebenden Gewebe starke Schmerzen oder gar Gewebsnekrosen und bleibende Schäden verursacht.

Dringt die Calciumhydroxid-Paste z.B. in den Mandibularkanal ein, kann es dort zu irreversiblen Schäden in Form von Nekrosen am n.mandibularis kommen. Die daraus resultierenden Beschwerden des Patienten sind außerdem erheblich.

Es hat sich somit gezeigt, daß eine exakte Tiefenbegrenzung bei der Applikation und dem Einbringen der Calciumhydroxid-Paste in den Wurzelkanal mit keiner der beiden Methoden erreichbar ist.

Außerdem gelingt das Abtöten der Keime nicht immer zufriedenstehend, denn besonders hartnäckige Mikroorganismen überleben nach derzeitigem Wissensstand sogar diese Behandlung und können bis zu 0,4 mm tief in Dentintubuli eindringen, um so als Monoinfektion zu überleben.

Als solche besonders hartnäckigen Mikroorganismen sind an sich nur Enterococcus faecalis und Candida albicans bekannt.

Dieser Mangel einer Behandlung mit Calciumhydroxid ist zwar in Fachkreisen grundsätzlich bekannt und diskutiert, wird aber derzeit in Ermangelung einer Alternative aufgrund einer fehlenden Alternative akzeptiert und hingenommen. Besonders im Hinblick auf den Enterococcus faecalis, der immer wieder im Wurzelkanal anzutreffen ist, hat sich gezeigt, daß Ca(OH)₂ nicht in der Lage ist, diesen Keim abzutöten.

Soweit bekannt, überlebt der Enterococcus faecalis nicht nur eine Behandlung mit Ca(OH)₂, sondern alle bisher bekannten keimtötenden Mittel (siehe z.B. Veröffentlichung von Estrela et.al, Int Endod J. 2007; 40:85-93).

Der Enterococcus faecalis ist darüberhinaus auch überdurchschnittlich resistent gegen Detergentien sowie hohe Salzkonzentrationen von > 6,5 % NaCl, wie z.B. in 1990 durch Publikationen von Dr. Safavi, University of Connecticut, als Ergebnis seiner Untersuchungen bekanntgeworden ist.

Neben diesem äußerst hartnäckigen Keim ist auch der des weiteren genannte Candida Albicans gegenüber CaOH wenig empfindlich (siehe z.B. Waltimo TM et al., Int Endod J. 1999 Nov;32(6):421-9).

Candida albicans gilt als der am häufigsten anzutreffende Keim, wobei dieser Pilz im Wurzelkanal in gleicher Häufigkeit wie in der übrigen Mundschleimhaut anzutreffen ist. Nach Borssen et al., 1981, ist der Candida albicans bei bis zu 40 % der Wurzelkanal-Infektionen zu finden. In vitro-Untersuchungen von Haapasalo et al. aus dem Jahr 2000 belegen darüber hinaus, daß dieser Pilz noch ein stark alkalisches Milieu, mit pH-Werten bis zu 12.5, toleriert (Waltimo TM et al., Oral Microbiol Immuno1.2000 Aug;15(4):245-8).

Bei einer Behandlung des Wurzelkanals wird die rein chemomechanischer Aufbereitung in Fachkreisen als "one visit treatment" und die Behandlung mit einer zusätzlichen medikamentösen Einlage als "two visit treatment" bezeichnet.

Nach Untersuchungen von Lan et al. aus dem Jahr 2004 lassen sich nach einer Behandlung mit der Methode des "one visit treatment" noch 62 % und selbst nach einer Behandlung mit der Methode des "two visit treatment" noch 27 % Bakterienwachstum nachweisen.

Im Vergleich dazu haben Studien von Vianna et al. aus dem Jahr 2006 gezeigt, daß auch das weiter oben genannte Chlorhexidin als ein alternatives Produkt nur 50 % der Keime im Wurzelkanal erreicht (Sena NT et al., Int Endodot J. 2006 Nov;39(11):878-85). Und auch aus anderen Gründen ist Chlorhexidin als chloriertes Produkt nicht unumstritten.

Ausgehend von diesem Stand der Technik lag der vorliegenden Erfindung daher die Aufgabe zugrunde, ein medizinisches Präparat bereitzustellen, das als Desinfektionsmittel insbesondere bei der Behandlung von Pulpengangränen eingesetzt werden kann, und daß insbesondere auch die bisher schwer oder gar nicht entfernbaren Mikroorganismen, wie Enterococcus faecalis und Candida albicans, abtötet, ohne in anderer Weise gesundheitsschädlich zu wirken.

Gelöst wird diese Aufgabe durch die erfindungsgemäße Verwendung von ozonisiertem Pflanzenöl und/oder dessen Bestandteilen oder von Jod, insbesondere PVP-Jod, als keimtötendes Mittel zur Herstellung eines medizinischen Präparats zur Behandlung der Zahnwurzel, insbesondere bei Gangränen.

Insbesondere auf der Grundlage des ozonisierten Pflanzenöls und/oder dessen Bestandteilen steht ein medizinisches Präparat zur Verfügung, das grundsätzlich dazu geeignet ist, alle Anaerobiet, zu denen auch der Enterococcus faecalis gehört, daneben viele Aerobier, alle Pilze/Dermatophyten sowie Viren zuverlässig abzutöten.

Als besonders vorteilhaft ist zu erwähnen, daß bei der Anwendung des erfmdungsgemäßen medizinischen Präparats jegliche Resistenzbildung ausgeschlossen ist. Darüber hinaus ist die Substanz vollständig und rückstandslos abbaubar, sowohl am Wirkort als auch in der Umwelt.

Als Bestandteile des ozonisierten Pflanzenöls, bei deren Einsatz ebenfalls die im Hinblick auf das Pflanzenöl selbst genannten Vorteile zum Tragen kommen, ist ebenso die Ölsäure zu nennen.

PVP-Jod wird handelsüblich auch als Povidon-Jod bezeichnet, mit einem Gehalt von 10% an verfügbarem Jod in der Lösung.

Gemäß einer Ausführungsform der erfindungsgemäßen Verwendung ist das ozonisierte Pflanzenöl ausgewählt aus Rapsöl, (auch Rüböl oder Rüpsenöl genannt), Sonnenblumenkernöl, Sojaöl, Rizinusöl, Olivenöl, Leinöl, Kokosöl, Palmöl, Teebaumöl sowie zumindest einem Bestandteil, Derivat und/oder Mischungen davon. Es soll aber an dieser Stelle darauf hingewiesen werden, daß es sich dabei um eine exemplarische Aufzählung der gängigen Pflanzenöle handelt und daß grundsätzlich jegliches Pflanzenöl für die Zwecke der vorliegenden Erfindung eingesetzt werden kann.

Das Derivat kann ein Ester sein, vorzugsweise ein Glycerinester einer einfach oder mehrfach ungesättigten Fettsäure.

Es ist hinlänglich bekannt, daß die natürlichen pflanzlichen Öle Glycerinester der höheren geradzahligen Fettsäuren, d. h. der Glyceride darstellen und bis zu ca. 97% aus Triglyceriden, bis zu ca. 3% aus Diglyceriden und bis ca. 1% aus Monoglyceriden aufgebaut sind. Tri-, Di- und Monoglyceride bestehen jeweils aus einem Molekül Glycerin, verestert mit 3 Molekülen, 2 Molekülen bzw. 1 Molekül Fettsäure. Die chemischen, physikalischen und biologischen Eigenschaften der Pflanzenöle werden von der Art der Fettsäurekomponente und ihrer Verteilung über die Triglyceridmoleküle bestimmt. Es ist außerdem hinlänglich bekannt, daß der Schmelzpunkt im allgemeinen mit zunehmendem Anteil an langkettigen Fettsäuren bzw. mit abnehmendem Anteil an kurzkettigen oder ungesättigten Fettsäuren steigt. Die Eigenschaften eines Triglycerids werden auch durch die Stellung der verschiedenen Fettsäuregruppen innerhalb des Triglyceridmoleküls bestimmt.

Die Fettsäuren selbst sind überwiegend geradzahlige, geradkettige, aliphatische Monocarbonsäuren mit Kettenlängen von C₄ bis C₂₄. Der Schmelzpunkt einer Fettsäure fällt mit abnehmender Kettenlänge und zunehmender Anzahl von Doppelbindungen. Pflanzenöle sind bei Zimmertemperatur flüssig wegen ihres erheblichen Anteils an ungesättigten Fettsäuren.

Wenn das jeweilige Pflanzenöl als Ester der jeweiligen mehrfach ungesättigten Fettsäuren eingesetzt wird, sind grundsätzlich die Methylester von besonderem Interesse. Hier sind insbesondere Rapsfettsäuremethylester, Rizinolsäuremethylester, Ölsäuremethylester, Linolsäuremethylester, Laurinsäuremethylester zu nennen, wobei diese Aufzählung lediglich exemplarisch ist. Die Möglichkeiten sind mannigfaltig und die Auswahl sowie die gegebenenfalls weitere Umsetzung des pflanzlichen Öls oder seiner Bestandteile mit weiteren Fettsäuren ist für den Fachmann aufgrund seines allgemeinen Fachwissens ohne weiteres möglich.

Ozonisiertes Pflanzenöl in Form von ozonisiertem Olivenöl ist bereits bekannt. Es wird als solches, in Kapseln oder in Form von Salben angeboten. Als Anwendungsgebiete werden äußerliche Einreibungen mit dem ozonisierten Olivenöl bei Gelenkschmerzen und Rückenschmerzen, multipler Sklerose und Akne genannt. Bei Neurodermitis und Psoriasis wird eine kombinierte äußerliche Behandlung mit kolloidalem Silber vorgeschlagen, wobei beides nacheinander verwendet wird.

Das Ozon weist üblicherweise aufgrund der Art seiner Herstellung in einem Ozongenerator Sauerstoff als Trägergas auf. Entsprechend weist das in dem Pflanzenöl enthaltene Ozon seinerseits Sauerstoff als Trägergas auf. Aufgrund dieses Sauerstoffgehalts ist es nicht erforderlich, dem ozonisierten Pflanzenöl ein Konservierungsmittel hinzuzufügen. Dasselbe gilt entsprechend für die Verwendung der Ölsäure, weiterer Bestandteile oder Derivate.

Zu einem gewissen Anteil werden Peroxide ausgebildet. Mit dem Gehalt an aktivem Sauerstoff, der in dieser peroxidischen Form gebunden ist, läßt sich die Wirksamkeit des ozonisierten Pflanzenöls bzw. die Wirksamkeit von dessen Bestandteilen noch steigern.

Die Erfindung betrifft auch das medizinische Präparat zur Behandlung der Zahnwurzel, insbesondere bei Gangränen, selbst, das in Form von vorzugsweise längenkalibrierten Papierspitzen eingesetzt wird, die eine Beschichtung mit ozonisiertem Pflanzenöl und/oder dessen Bestandteilen bzw. Derivaten aufweisen, wie weiter oben bereits erläutert, oder eine Beschichtung in Form von Jod, insbesondere PVP-Jod.

Über längenkalibrierte Papierspitzen wird mittels der Methode der Endometrie eine genaue Platzicrung der Papierspitzen möglich. Die Methode der Endometrie beruht auf der Tatsache, daß der elektrische Widerstand zwischen der Mundschleimhaut und dem Desmodont konstant ist. Daraus wird abgeleitet, daß der Widerstand auch dann in einem konstanten Verhältnis stehen muß, wenn eine Meßsonde mit dem apikalen Desmodont via Wurzelkanal in Berührung kommt. Wird das Wurzelkanalinstrument als eine Elektrode in den Wurzelkanal eingeführt, wobei sich die Referenzelektrode an der Mundschleimhaut befindet, so besteht ein meßbarer Widerstand. Neuartige Meßinstrumente zur Erfassung der Wurzelkanallänge (Endometer) nehmen eine Relativmessung anhand der Impedanzen bei verschiedenen Meßfrequenzen vor. Über diese Methode der elektronischen Längenbestimmung der Wurzelkanäle kann anhand der Längenkalibrierung an den Papierspitzen durch einfaches Ablesen genau festgelegt werden, wie weit eine solche Papierspitze in den Wurzelkanal einzuführen ist.

Die Erfindung betrifft des weiteren die Verwendung des zuvor beschriebenen medizinischen Präparats als temporäre Einlage in das Endodont

Ganz besonders, aber keineswegs ausschließlich, eignet sich das zuvor beschriebene medizinische Präparat zur Abtötung von Keimen, ausgewählt aus dem besonders hartnäckigen Bakterium Enterococcus faecalis und dem Pilz Candida albicans.

Im folgenden soll die Erfindung anhand von Ausführungsbeispielen näher erläutert werden.

### Beispiel 1:

### L Beschreibung der Papierspitze (Paper Tip)

Für die Zwecke dieser Versuchsdurchführung wurden Papierspitzen verwendet, wie sie handelsüblich als Dentalbedarf für die Wurzelbehandlung erhältlich sind. Es wurden verschiedene Papierspitzen verwendet, die jeweils ca. 29 mm Länge aufwiesen, und folgenden Normen genügten:
- ISO 120,
- ISO 130,
- ISO 140,
- ISO 15-40 und
- ISO 45-80.

Allen verwendeten Papierspitzen gemeinsam war, daß sie aus feinstem Japanpapier hergestellt und stark saugend waren. Sie wurden maschinell hergestellt und konfektioniert. Die verwendeten Papierspitzen als solche waren alle ein reines Naturprodukt, die ohne ein Bindemittel oder sonstige chemische Zusätze gerollt wurden. Sie sind grundsätzlich formstabil und bewahren ihre Formstabilität im wesentlichen auch im feuchten Zustand.

### II. Präparierung des Trägers

Die unter I. beschriebenen Papierspitzen wurden mit ozonisiertem, d.h. mit Ozon angereichertem Olivenöl beschichtet. Trägergas für das Ozon ist Luftsauerstoff. Dazu wird das durch die Ozonisierung leicht pastöse Olivenöl dünn und vollflächig auf der Außenseite der Papierspitze aufgebracht, indem die Papierspitzen in einen die Paste aufweisenden Behälter langsam eingeführt und anschließend wieder herausgezogen wurden. Auf diese Weise wird bereits das fertige medizinische Präparat erhalten.

Es ist allerdings an dieser Stelle darauf hinzuweisen, daß die Herstellung des medizinischen Präparats hier für die Zwecke der die Durchführbarkeit und den Erfolg der die Erfindung erprobenden Laborversuche beschrieben worden ist.

Um die das medizinische Präparat bildenden, erfindungsgemäß imprägnierten Papierspitzen in einem größeren bis großem Maßstab herzustellen, können andere, den Erfolg ebenso herbeiführende, das Prinzip jedoch nicht verlassende Herstellungsmethoden verwendet werden. Insbesondere kann dann vorgesehen sein, das medizinische Präparat fertig, in Folie vorzugsweise aseptisch eingeschweißt, für die breite Anwendung zur Verfügung zu stellen.

### III. Einsetzen der Papierspitzen als temporäre Einlage in das Endodont

Bei fünfzehn ausgesuchten, freiwilligen Testpersonen (Probanden), die mit Anzeichen einer schmerzhaften Pulpitis in die Behandlung kamen, wurde der befallene Zahn zunächst geröntgt, um nähere Informationen über das Pulpengangrän und die notwendigen grundsätzlichen Informationen über die anatomischen Gegebenheiten im Wurzelbereich zu erhalten. Diese für eine erfolgreiche Behandlung erforderliche Maßnahme dient gleichzeitig der Dokumentation der erfindungsgemäßen Behandlung.

Mit einem sterilen Rosenbohrer wurde dann ein Zugang zu dem Zahnmark freigelegt und die entzündete Kronenpulpa mit einem Exkavator entfernt. Anschließend wurde die Kanalpulpa mit einer Pulpanadel entfernt und die Känale wurden gereinigt, gegebenenfalls leicht erweitert.

Nach jedem Arbeitsschritt wurden die Wurzelkanäle mit einer desinfizierenden Lösung ausgespült und mit den bereits oben, unter I. beschriebenen, jedoch noch nicht erfindungsgemäß vorbereiteten Papierspitzen getrocknet. Damit wurden sie für eine erfindungsgemäße, temporäre Wurzelfüllung vorbereitet. Gleichzeitig wurden die Papierspitzen für eine bakteriologische Untersuchung verwendet, um die Art des bakteriellen Befalls und den Erfolg der temporären Einlagen feststellen zu können. Tatsächlich wurde bei neun der Probanden ein bakterieller Befall unter anderem mit "Enterococcus faecalis" diagnostiziert.

Der jeweilige Wurzelkanal wird nun mit der temporären Wurzelfüllung abgefüllt, indem erfindungsgemäß, wie unter I. beschrieben präparierte Papierspitzen in den jeweiligen Kanal gepreßt und soweit nachgeschoben werden, bis der Kanal im wesentlichen dicht gefüllt ist. Diese desinfizierende Füllung kann nun Tage bis wenige Wochen zur Entkeimung des Wurzelkanalsystems an Ort und Stelle belassen werden.

Bei den hier vorgestellten fünfzehn Probanden wurde die Wurzelfüllung mittels der Papierspitzen etwa eine Arbeitswoche in dem Wurzelkanalsystem belassen, dann entfernt und die Reste der entfernten Papierspitzen wieder bakteriologisch untersucht.

Es konnte in allen behandelten Fällen im wesentlichen Keimfreiheit festgestellt werden.

Anschließend wurden die Wurzelkanäle mittels üblicher lateraler Kondensation gefüllt und verschlossen. Die behandelten Patienten wurden anschließend röntgenologisch überwacht. Sie blieben im weiteren Verlauf beschwerdefrei und ohne Befund.

Zum Vergleich wurde eine entsprechende Anzahl von Probanden mit Ca(OH)₂ in herkömmlicher Weise behandelt.

Im Gegensatz zu der Behandlung mit Papierspitzen, welche mit ozonisiertem, d.h. mit Ozon angereichertem Olivenöl beschichtet waren, ergab die Verwendung einer Einlage mit Calciumhydroxid eine Erfolgsrate von 74 %.

### IV. Präparierung des Trägers mit ozonisierter Ölsäure

Gemäß dieser Variante wird nicht ozonisiertes Olivenöl für die Herstellung des medizinischen Präparats verwendet und der Träger damit beschichtet, sondern ozonisierte Ölsäure. Trägergas für das Ozon ist wieder Luftsauerstoff.

Ölsäure oder Oleinsäure C₁₇H₃₃COOH findet sich z.B. in Form gemischter Glycerinester in großer Menge in vielen pflanzlichen Ölen. Im Oliven- und Mandelöl ist sie als Hauptbestandteil zu nennen.

Für die Zwecke der vorliegenden Erfindung wird die reine freie Ölsäure in die Definition eines Bestandteiles der jeweiligen Pflanzenöle einbezogen. Aufgrund ihrer Löslichkeit in DMSO wurde die Ozonisierung der Ölsäure unter anderem in einer Lösung in DMSO durchgeführt. Das erhaltene Produkt wies eine gegenüber dem ozonisierten Pflanzenöl etwas flüssigere Konsistenz auf.

### V. Präparlerung des Trägers mit kalibrierten Papierspitzen

Es wurden Papierspitzen, wie sie von der Firma RÖKO handelsüblich erhältlich sind und als solche bisher zum Trocknen der Kanäle benutzt werden, gemäß einer Variante mit ozonisiertem, d.h. mit Ozon angereichertem Olivenöl beschichtet. Die Papierspitzen wiesen eine Längenkalibrierung auf, indem auf ihnen Längenabschnitte markiert waren. Trägergas für das Ozon ist Luftsauerstoff, Dazu wird das durch die Ozonisierung leicht pastöse Olivenöl dünn und vollflächig auf der Außenseite der Papierspitze aufgebracht, indem die Papierspitzen in einen die Paste aufweisenden Behälter langsam eingeführt und anschließend wieder herausgezogen wurden. Auf diese Weise wird bereits das fertige medizinische Präparat erhalten.

Gemäß einer anderen Variante wurde ozonisierte Ölsäure, wie unter IV. beschrieben, für die Präparierung des Trägers in Form der konfektionierten, kalibrierten Papierspitzen verwendet. Da die ozonisierte Ölsäure nach der Ozonisierung im Vergleich zu dem nach der Ozonisierung eher pastösen Olivenöl flüssiger erhalten wird, eignet sie sich gut für die Applikation auf einer Papierspitze. Die Papierspitze wird zu ihrer Imprägnierung oder Beschichtung in die ozonisierte Ölsäure eingetaucht. Es wird ein dünner und vollflächiger Belag auf der Außenseite der Papierspitze aufgebracht, indem die Papierspitzen in einen die ozonisierte Ölsäure aufweisenden Behälter langsam eingeführt und anschließend wieder herausgezogen wurden. Die Ölsäure kann dabei auch zumindest teilweise in die Papierspitze einziehen.

Durch die Anwendung der längenkalibrierten Papierspitzen in Verbindung mit der Endometrie ergibt sich eine bessere Handhabung und zugleich eine erhöhte Anwendungssicherheit. Die jeweilige Papierspitze kann exakt appliziert werden, ein Überstopfen über den Apex wird dadurch verhindert. Es ist jeweils deutlich die individuell ganz verschiedene Eindringtiefe für jede Wurzelbehandlung feststellbar.

Eine exakte Längenpositionierung, welche das Überstopfen wirksam verhindert, bedeutet dabei keinen wesentlichen Mehraufwand.

Über das Endometer wird schon bei der Aufbereitung die Länge jedes einzelnen Kanals elektronisch präzise abgemessen, wie weiter oben beschrieben. In dieser Weise kann - genau wie bei der späteren definitiven Abfüllung - die Länge der mit dem erfindungsgemäßen Präparat beschichteten Papierspitzen genau abgemessen und kontrolliert werden. Die Papierspitze wird entsprechend der vorher abgemessenen Länge bis kurz vor dem Apex platziert und hinterher auch wieder problemlos entfernt, um so ein Überstopfen der Einlage sicher und zuverlässig vermeiden.

Insgesamt kann daher zusammenfassend festgestellt werden, daß zum einen der Vorteil des erfindungsgemäßen medizinischen Präparats gegenüber einer eingerührten oder eingestopften Calciumhydroxid-Einlage in der breiteren antimikrobiellen Wirkung besteht, die vor allem den Enterococcus faecalis einbezieht, und zum anderen auch eine sichere Wirkung gegen Candida albicans bzw.gegen Pilze allgemein erreicht werden kann.

### Beispiel 2:

Auch in diesem Beispiel wurde in allem genauso verfahren, wie weiter oben in Beispiel 1, unter I. - II. angegeben. Die Papierspitzen gleicher Art wurden in diesem Beispiel aber nicht mit Ozon angereichertem Pflanzenöl, sondern mit PVP-Jod", auch "Povidon-Jod"genannt, durch ein Tauchbad imprägniert. Unter Povidon-Jod ist ein Poly[1-vinyl-2-pyrrolidon]-Jod-Komplex zu verstehen. 100 ml der eingesetzten Lösung enthalten 10 g Povidon-Jod mit einem mittleren Molekulargewicht von 44.000 und mit einem Gehalt von 10% an verfügbarem Jod.

Es wurden den Untersuchungen drei, jedoch von den Probanden, wie unter II. angegeben, verschiedene Probanden zugrundegelegt. Bei der Auswahl der Probanden wurde so lange experimentiert, bis zumindest einer der Probanden einen Befall mit dem besonders hartnäckigen und resistenten "Enteroccocus fäcalis" zeigte.

Die bakteriologischen Ergebnisse waren im wesentlichen dieselben, wie in Beispiel 1 beschrieben.

Damit konnte gezeigt werden, daß sowohl ozonisiertes Pflanzenöl als auch Jod, insbesondere PVP-Jod, als keimtötendes Mittel zur Behandlung von Pulpengangränen geeignet sind und deutlich bessere Resultate hervorbringen, als dies bisher im Stand der Technik möglich ist.

Neben den genannten Probanden, deren Laboruntersuchungen für die Zwecke dieser Beispiele genauer analysiert und dokumentiert worden sind, gibt es inzwischen eine Reihe von weiteren Probanden, die unter Verschwiegenheit mit ozonisiertem Pflanzenöl, ozonisierter Ölsäure und PVP-Jod als keimtötendem Mittel im Wurzelbereich behandelt wurden. Bei allen Probanden konnte eine erfolgreiche Behandlung ohne Rezidiv durchgeführt werden.

Insbesondere wurden weitere Probanden bei der Behandlung mit PVP-Jod als keimtötendem Mittel danach ausgesucht, ob sie sich anfällig für Allergien zeigten oder nicht. Dabei wurden - unter vorheriger ausführlicher Aufklärung - besonders solche freiwilligen Patienten mit PVP-Jod als keimtötendem Mittel behandelt, die sehr allergieanfällig waren. Sie haben die Behandlung ohne jegliche Nebenwirkungen und/oder Komplikationen überstanden. Allergische Reaktionen konnten bisher in keinem Fall beobachtet und festgestellt werden.

Die Anwendung zeichnet sich somit insbesondere dadurch aus, daß sie äußerst effizient, dabei einfach, für den betroffenen Patienten ohne zusätzliche Schmerzen und Mühe und vor allem kostengünstig durchführbar ist. Dies gilt gleichermaßen für die Anwendung von ozonisiertem Pflanzenöl, ozonisierter Ölsäure als auch PVP-Jod als keimtötendem Mittel im Wurzelbereich.

## Patentansprüche

1. Verwendung von ozonisiertem Pflanzenöl und/oder dessen Bestandteilen oder von Jod, insbesondere PVP-Jod, als keimtötendem Mittel zur Herstellung eines medizinischen Präparats zur Behandlung der Zahnwurzel, insbesondere bei Gangränen.

2. Verwendung nach Anspruch 1, **dadurch gekennzeichnet, daß** der Bestandteil des ozonisierten Pflanzenöls Ölsäure ist.

3. Verwendung nach Anspruch 1, **dadurch gekennzeichnet, daß** das ozonisierte Pflanzenöl ausgewählt ist aus Rapsöl, Sonnenblumenkernöl, Sojaöl, Rizinusöl, Olivenöl, Leinöl, Kokosöl, Palmöl, Teebaumöl, zumindest einem Bestandteil, Derivat und/oder Mischungen davon.

4. Verwendung nach Anspruch 3, **dadurch gekennzeichnet, daß** das Derivat ein Ester ist, vorzugsweise ein Glycerinester einer einfach oder mehrfach ungesättigten Fettsäure.

5. Verwendung nach einem der Ansprüche 1 bis 4, **dadurch gekennzeichnet, daß** das in dem Pflanzenöl, dem Bestandteil oder Derivat des Pflanzenöls enthaltene Ozon seinerseits Sauerstoff als Trägergas aufweist.

6. Medizinisches Präparat zur Behandlung der Zahnwurzel, insbesondere bei Gangränen, in Form von Papierspitzen, die eine Beschichtung von ozonisiertem Pflanzenöl und/oder dessen Bestandteilen nach einem der Ansprüche 1 bis 5 aufweisen.

7. Medizinisches Präparat zur Behandlung der Zahnwurzel, insbesondere bei Gangränen, in Form von Papierspitzen, die eine Beschichtung in Form von Jod, insbesondere PVP-Jod, nach Anspruch 1 aufweisen.

8. Medizinisches Präparat zur Behandlung der Zahnwurzel nach Anspruch 6 oder 7 in Form von längenkalibrierten Papierspitzen.

9. Verwendung des medizinischen Präparats nach einem der Ansprüche 6 bis 8 als temporäre Einlage in das Endodont.

10. Verwendung nach Anspruch 9 zur Abtötung von Keimen, ausgewählt aus Enterococcus faecalis und Candida albicans.
